# EUROPEAN PATENT SPECIFICATION

(11) **EP 3 756 005 B1**
(45) Date of publication and mention of the grant of the patent: **22.09.2021**
(21) Application number: 19704686.5
(22) Date of filing: 11.02.2019
(51) Int. Cl.: G01N 33/00, F04B 49/00

(54) **DEVICE FOR MONITORING THE QUALITY OF THE COMPRESSED AIR AND ASSOCIATED METHOD**
VORRICHTUNG ZUR ANZEIGE DER QUALITÀT VON KOMPRIMIERTER LUFT UND VERFAHREN DAZU
DISPOSITIF DE SURVEILLANCE DE LA QUALITÉ D'AIR COMPRIMÉ ET PROCEDÉ ASSOCIÉ

(30) Priority: 20.02.2018 BE 201805105
(43) Date of publication of application: 30.12.2020
(73) Proprietor: ATLAS COPCO AIRPOWER, naamloze vennootschap, 2610 Wilrijk (BE)
(72) Inventor: TALBOOM, Bart, 2610 Wilrijk (BE)
(74) Representative: Van Minnebruggen, Ewan Benito Agnes
(86) International application number: PCT/IB2019/051076
(87) International publication number: WO 2019/162795

(56) References cited:
- EP-A1- 3 079 109
- WO-A1-01/82063
- WO-A1-2005/015175
- WO-A1-2012/059425
- WO-A1-2013/019178
- WO-A1-2016/135436
- WO-A2-02/066973
- DE-B3-102005 059 304
- US-A- 4 267 030

## Description

The present invention relates to a device for monitoring the quality of the compressed air from a compressed air network and calibrating a measuring device used therein.

Some industrial applications use compressed air that has to meet high quality standards in the field of purity, whereby more specifically only a low concentration of impurities is required.

Such critical applications are typically known in the pharmaceuticals sector, the food and beverage sector, the electronics industry, high-quality medical precision tool manufacturing, and the like.

More generally, compressed air is subject to high quality standards in any industry where the use of impure air poses a potential hazard to the production process or contamination of the end product.

It is known that quality audits are regularly conducted in these critical sectors.

However, in some sectors, even in the case of frequent audits, there remains a significant potential risk, for example in the field of liability in the event of products entering the market that are contaminated by contact with impurities in the compressed air, or in the case of a high risk of contaminated products having to be scrapped or recalled from the market, etc.

In such cases, the quality of the compressed air used must be continuously monitored by measuring the presence or absence of specific impurities in the air.

In this context, standard ISO 8573 was created in order to determine a quality class of compressed air in an unambiguous and uniform way by measuring solid particles in the compressed air and/or by measuring the pressure dew point as a measure for the presence of water in the compressed air and/or by measuring the presence of oil in the form of droplets or vapour.

Measuring devices to measure particles, the dew point and the presence of oil can be found on the market, which are intended mainly for measurement under laboratory conditions, but are not suitable for industrial use as intended in this application.

One of the requirements in case of industrial use is a regular calibration of the measuring devices, which is a challenge in case the compressed air quality has to be continuously monitored with the measuring devices.

One possibility is to regularly return the measuring devices to the manufacturer or to a service centre for calibration. In this case, there is a considerable period of time during which no measurement of the compressed air quality is possible, unless a replacement device is used while the original device is being calibrated.

For this reason, some suppliers provide an exchange service whereby the device requiring calibration is replaced by a calibrated replacement device which can then remain in service until this replacement device requires calibration.

In the meantime, the measuring device to be calibrated is sent to the supplier and, when it returns calibrated, is stored in a warehouse of replacement devices.

Other manufacturers or service providers have established a service to perform on site calibration at the compressed air network site whereby a technician is sent on site to perform a calibration. For dew point and oil, this calibration is performed with test gases of a known impurity level to confirm the required accuracy of the measuring device or to adjust the device if necessary.

These test gases or so-called "span gases" are gases with a precisely known concentration of a certain impurity for which the measuring device to be calibrated is intended and the concentration of which is close to the maximum range of the measuring device to be calibrated or also contain a so-called zero gas without any contamination in it.

These test gases are supplied in gas cylinders with a certificate to ensure that the certified impurity is correct within a specified accuracy.

A homogeneous mixture of particles in air cannot be obtained for the measurement of solid particles. For this reason, particle measurements are carried out using a solution of solid particles in a liquid. This liquid is sprayed, vaporised and then presented to the particle counter as a homogeneous gas-particle mixture during the calibration process. In the further explanation no distinction is made between a test gas that is supplied in cylinders or a test gas that is obtained from the spraying and vaporising of a test liquid.

A disadvantage is that such on site services are expensive, as a technician has to be sent on site, has to have access to the compressed air network, has to prepare the necessary equipment and the like. In many cases, the extra cost of the journeys and the actions, apart from the calibration, is much greater than the cost of the calibration itself.

The document DE 10 2005 059304 discloses a method for calibrating a humidity sensor using a gas with known humidity concentration.

The purpose of the present invention is to provide a solution to one or more of the aforementioned and other disadvantages.

To this end, the invention relates to a device for monitoring the quality of the compressed air in a supply line of a compressed air network and calibrating a measuring device used for this purpose, more specifically a measuring device for measuring the quantity of a specific type of impurity in the compressed air, whereby the device is provided with:
- a measuring line which is intended to be connected to the supply line and which is provided with a shut-off valve to close the measuring line of the supply line;
- an aforementioned measuring device with an inlet for gas which is connected to the measuring line and a blow-off valve;
- means for supplying a test gas flow with a known impurity, more specifically with a known concentration or number of particles of the specific impurity, connected to the measuring line (8, 8') via a controllable shut-off valve (15) to enable the test gas to pass through it;
- a control (19) to which the aforementioned shut-off valves (10, 16) are connected to control these shut-off valves (10, 16) in such a way that either compressed air can be sent through the measuring device for monitoring the quality of the compressed air, or an aforementioned test gas can be sent through the measuring device to calibrate the measuring device.

Therefore, by integrating the means for supplying the test gases, whether in the form of gas cylinders containing test gases or in the form of a test solution with spray and vaporiser, into the device and providing the necessary shut-off valves and controls to periodically, or whenever deemed necessary, carry out a calibration cycle with one or more test gases, the whole calibration process can be fully automated for one or more measuring devices with a minimum loss of time for the continuous monitoring of the compressed air quality due to the unavailability of one or more measuring devices.

The device is easy to install in any existing compressed air network by connecting the measuring line to a supply line of the compressed air to be monitored of the compressed air network.

Preferably, the control is programmed in such a way that for each measurement with a test gas the measuring line is first flushed with a quantity of zero gas and then with a quantity of the test gas concerned which is passed through it before closing the measuring line to carry out the calibration.

Flushing ensures that no residual impurities of the test gas remain in the measuring line used for a previous calibration or from the compressed air to be monitored which passed through it before.

Practically, it is preferable that the device contains the means to allow a dosed quantity of test gas to pass to the measuring line for each calibration measurement.

Thus, the content of a new gas cylinder tells us exactly how many calibrations can be made with it before the gas cylinder is empty and it also prevents waste of the expensive test gas.

According to a preferred characteristic the device is equipped with several measuring devices connected to the measuring line, each measuring device being intended for the measurement of the concentration of a specific type of impurity, including for example at least one of the following measuring devices or a combination thereof:
- a measuring device to measure the concentration of solid particles;
- a measuring device to measure the humidity, for example in the form of a device to measure the pressure dew point;
- a measuring device to measure the concentration of oil, whether in liquid or vapour form

With such a device according to the invention it is possible to automatically assign a quality class to the compressed air based on the continuous measurement results of the calibrated measuring devices, for example based on the application of the ISO 8573 standard.

By linking the device to a monitoring system to collect and record the measurement data and the calibration data of each measuring device and other data such as, for example, the lot number of the used and in use gas cylinders as well as the pressure and temperature, it can be consulted at any time and quality certificates can be automatically generated for which the services of third parties must otherwise be used.

For example, the monitoring system can make the data available to the user and the supplier of the compressed air network via a cloud service, so that the quality of the supplied compressed air is guaranteed, including the generating of the necessary certificates without having to go on site every few months to perform a calibration. An on-site visit every few years should be sufficient.

In terms of proof of liability, it is also useful to be able to trace historical data.

The invention also relates to a compressed air network which contains a device according to the invention for monitoring the quality of the compressed air from the compressed air network and for the automatic calibration of a measuring device used for this purpose.

The invention also relates to a method for monitoring the quality of the compressed air from a compressed air network, using at least one measuring device to measure the concentration of a specific impurity and to periodically calibrate the used measuring device connected to a measuring line, characterised in that the method contains the following steps:
- during the periods that the quality of the compressed air is monitored, branching off a compressed air flow to be monitored via the measuring line to pass it through the measuring device and continuously or periodically measuring the concentration of the relevant impurity in the compressed air to be monitored;
- during the periods of periodic calibration of the measuring device used, isolating the measuring line with the measuring device from the compressed air system and performing the following steps:
   - providing a test gas of a known concentration of the specific impurity for which the measuring device is intended and the provision of a test gas flow which is passed via the measuring line through the measuring device to be calibrated;

   - measuring the concentration of this test gas and determining the deviation of the measured value compared to the known value of the test gas in question;
   - where appropriate, correcting the measurement value of the measuring device as a function of the detected deviations and/or recording the calibration data in a monitoring system.

With the intention of better showing the characteristics of the invention, hereinafter, by way of an example without any limiting nature, a number of preferred embodiments are described of a device according to the invention for monitoring the quality of the compressed air from a compressed air network and calibrating a measuring device used therein, and of a method applied thereby, with reference to the accompanying drawings, wherein:
figure 1 schematically shows a device according to the invention integrated in a compressed air network;
figure 2 shows a flow diagram of a method according to the invention;
figures 3 and 4 each show a possible variant of a device according to the invention.

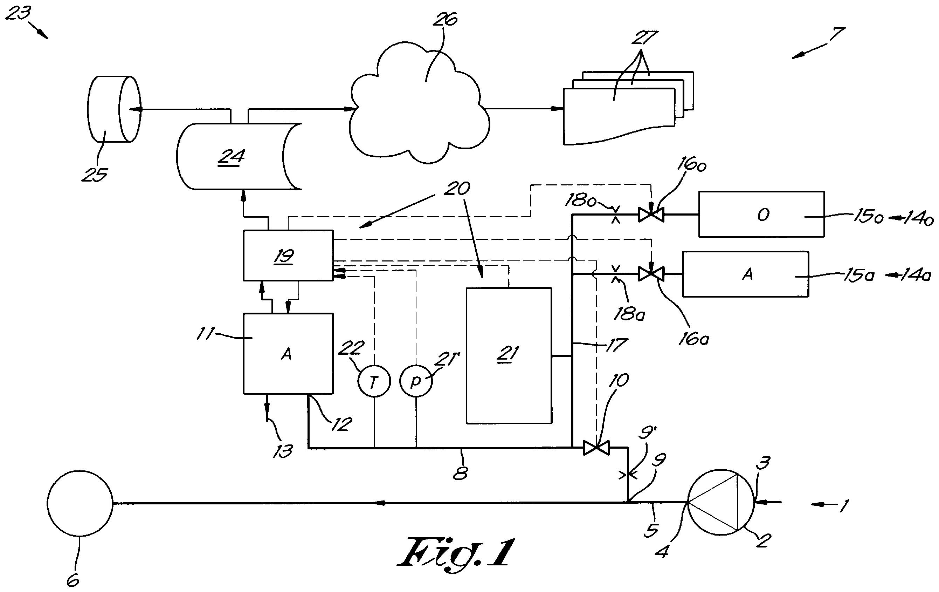

As is customary, the compressed air network 1 shown in figure 1 contains a compressor 2 with an inlet 3 and an outlet 4 and a supply line 5 connected thereto for supplying compressed air to a consumer network 6 with specific quality requirements for the supplied compressed air.

The compressed air network 1 contains a device 7 according to the invention for the permanent or periodic monitoring of the quality of the supplied compressed air.

To this end, the device 7 contains a measuring line 8 with an entry 9 connected to the aforementioned supply line 5 at a branch point to branch off a leakage flow from the compressed air to be monitored and provided for this purpose with means to limit or control the branched off flow, for example, means 9' in the form of a calibrated passage.

The measuring line 8 is also provided with a controlled shut-off valve 10 downstream from its entry 9, which allows the measuring line 8 to be closed from the supply line 5.

A measuring device 11 is included in the measuring line 8 downstream from the shut-off valve 10, at least the sensor of such measuring device 11, with an inlet 12 and a blow-off valve 13 which also forms an outlet of the measuring line 8 which, in the case of the figures, blows into the environment.

The measuring device 11 is intended for measuring the concentration of a specific impurity that would be present in the compressed air that is led through the measuring device 11 via the measuring line 8 to monitor its quality continuously or periodically.

The sensor of the measuring device is not shown separately but forms a component of the measuring device 11.

The measuring device 11 is, for example, a measuring device to measure the number of solid particles or to measure the pressure dew point with respect to the humidity of the compressed air or to measure the concentration of oil in droplet or vapour form.

The measured values can allow a quality class to be assigned to the supplied compressed air, for example based on the ISO standard 8573.

The quality class in ISO 8573 is expressed as ISO8573 [A:B:C], in which A specifies the class of the maximum number of solid particles per volume unit as a function of particle size, B specifies the class with respect to the pressure dew point and thus with respect to humidity and C specifies the class with respect to the oil concentration in the compressed air.

The value of A, B and C is expressed with a number. The smaller the value of the number, the better the quality of the compressed air.

Depending on the intended industrial application of the compressed air, not all three values A, B and C are evaluated. It may be sufficient that only one or two values determine the quality of the compressed air for that application.

The device 7 according to the invention shown in the example in figure 1 also contains means 14 to supply a certain test gas flow, respectively:
- means 14o for the supply of a test gas in the form of a so-called pure zero gas free of type A impurities for which the measuring device 11 is intended, in particular free of any type A, B and C impurities as defined in ISO 8573; and,
- means 14a for the supply of an impure test gas of a known concentration of the type A impurity, more specifically in the form of solid particles.

For the sake of clarity, it is assumed that the measuring device in figure 1 is intended to measure impurities A in the form of solid particles.

In the example shown in figure 1, the means 14o and 14a are formed by a gas cylinder 15, 15o and 15a respectively, filled with the respective test gas, which is connected to the measuring line via a shut-off valve 16, 16o and 16a respectively, and a line 17, more specifically at a point between the shut-off valve 10 and the measuring device 11, in order to be able to pass test gas flow through the measuring device 11 when the shut-off valve 10 is closed.

The means 14 may also be provided with means 18, 18o and 18a respectively, to control the supplied test gas flow, for example in the form of a flow regulator, a calibrated passage or any other suitable alternative.

Instead of a gas cylinder 15 filled with test gas, for example, a receptacle containing a test liquid combined with a spray or a vaporiser can be used to spray or vaporise the test liquid in a supplied zero gas flow.

The device 7 also contains a control 19 with which the aforementioned shut-off valves 10 and 16 are connected for the automatic control of these shut-off valves 10 and 16, whereby the control is programmed in such a way that:
- during periods of compressed air quality monitoring, the measuring line 8 is opened by the control of the shut-off valves 10 when the shut-off valves 16 are closed to allow the compressed air to be monitored to flow through the measuring device 11 for continuous or periodic measurement of the purity of the compressed air with closed shut-off valves 16; or,
- during a calibration of the measuring device 11, selectively and alternately providing the measuring line 8 with a test gas from one of the cylinders 15o or 15a to check whether there is a deviation between the value measured by the measuring device 11 and the known value of the concentration of impurity A of the zero gas 0 or of the test gas.

Preferably, the measuring device 11 shall be one the measurement result of which can be corrected for calibration reasons and control 19 is programmed to correct the measurement results of the measuring device 11 according to a determined deviation of the measurement result compared to the known value of the concentration of impurity A in the measured test gas.

In that case, a two-way communication between the measuring device 11 and the control 19 shall be possible, more specifically in one way to read the measurement results and in the other way to correct the measurement values for calibration purposes.

The device 7 is further provided with dosing means 20 to allow a dosed quantity of test gas to pass to the measuring line 8 for each calibration measurement, for example in the form of a flow meter 21 in combination with the control 19 programmed for this purpose to open and close in doses the shut-off valve 16 of a relevant gas cylinder 15 with test gas.

Alternatively, this can also be achieved by a pressure measurement of the test gas using a pressure gauge 21' or any other alternative.

A temperature gauge 22 allows the temperature of the test gas to be determined, as this too can influence the dosage of the test gas.

The device 7 also contains a monitoring system 23 with a data acquisition system 24 to collect and record the measurement data and calibration data of the measuring device 11.

The data acquisition system 24 can be part of the user's management system 25 for permanent access to the stored data or can be part of a cloud service.

The monitoring system 23 also keeps the data of used and in use test gas cylinders, for example with their lot number and the period during which they have been in use, all this in the context of the required traceability.

The user of the compressed air network or the supplier of the compressed air network or any third party authorised to do so is able to consult the data of the monitoring system 23 at any time.

The use of the aforementioned device 7 according to the invention is simple and as follows.

To monitor the compressed air from the compressed air network 1, the shut-off valve 10 is opened while the shut-off valves 16 are closed, all this to allow a leak flow of the monitored compressed air to flow through the measuring device 11, which leak flow is blown off in the free atmosphere.

The measuring device 11 is then used to permanently or periodically measure and record the concentration of impurity A in the compressed air.

Based on the measurement results, a quality class can be assigned, for example based on the ISO 8573 standard.

In this case, for example, only the number of solid particles is measured and the quality class will only be determined as class ISO8573 [A:-:-] as a function thereof.

The control 19 is programmed such that the measuring device 11 is automatically periodically calibrated.

In this case, the shut-off valve 10 at the entry 9 of the measuring line 8 is closed such that the measuring line is isolated from the compressed air network 1.

The measuring line is then flushed with a dosed quantity of zero gas, for which the shut-off valve 16o is opened for a certain time. The zero gas then flows through the measuring device 11 and is blown off in the free atmosphere.

A calibration measurement is then performed with the measuring device 11.

If the measuring device 11 is working correctly, the measurement result should indicate that no solid particles are being measured, as it is known that the zero gas is guaranteed to not contain solid particles.

If the measurement result deviates from this, the measuring device 11 can be corrected to obtain a zero measurement value, at least if the measuring device is suitable for this purpose. The measured deviation and the applied correction are recorded.

A calibration shall then be performed with the test gas A as follows.

First the shut-off valve 16a is opened to flush the measuring line 8 with test gas A to make sure there is no more zero gas in the measuring line 8.

Subsequently, a measurement is performed with the test device 11, the result of which, when the test device 11 is operating correctly, should give a value corresponding to the known number of solid particles of the test gas A.

If a deviation is found, the measuring device shall be adjusted if equipped to do so.

The measurement results and any corrections are recorded to finalise the calibration.

It is then possible to switch back to the normal operation by closing the shut-off valve 16a again and opening the valve 10 of the measuring line 8 to send the compressed air through the measuring line 8 again to continuously or periodically measure the concentration of solid particles present in the compressed air.

Measurements are only started when sufficient compressed air has flowed through the measuring line to ensure that the test gas from a previous calibration measurement has been completely flushed out of the measuring line 8 to guarantee a stable measurement. The delay of the restart of the monitoring depends on the method of installation of the device 7, among other things on the diameter and length of the lines between the gas cylinders 15 and the measuring device 11, on the test gas flow to the measuring device 11, on the compressed air flow to be monitored and the like.

The monitoring system may be equipped to generate a quality certificate based on all measurement data, calibration data such as deviations and corrections and data relating to the traceability of the test gases used. This allows a total quality policy to be pursued.

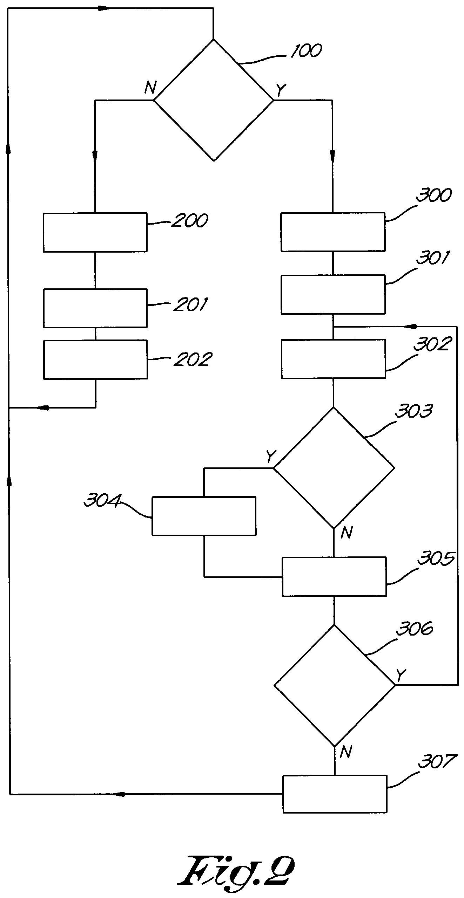

Figure 2 schematically shows the flow diagram of the method described above, with the following steps:
- in step 100 find out whether a periodic calibration is required or not;
- if in step 100 no calibration is required, continuing the monitoring 100 with the following steps:
   ∘ step 200: sending a compressed air flow to be monitored through the measuring device 11;
   ∘ step 201: measuring impurity A;
   ∘ step 202: recording the measurement result;
- if calibration is required in step 100, performing a calibration cycle with the following steps:
   ∘ step 300: isolating the measuring device 11 from the compressed air network;
   ∘ step 301: sending a test gas flow through the measuring device;
   ∘ step 302: performing a measurement with the test device 11;
- step 303: determining whether there is a deviation between the measured impurity and the known impurity of the test gas;
   ∘ if in step 303 a deviation is determined, possibly adjusting the measuring device in step 304;
- step 305: recording the calibration data;
- step 306: possibly repeating steps 302 to 305 with another test gas and in this case flushing the measuring device 11 with zero gas;
- step 307: generating a quality certificate and returning to step 100.

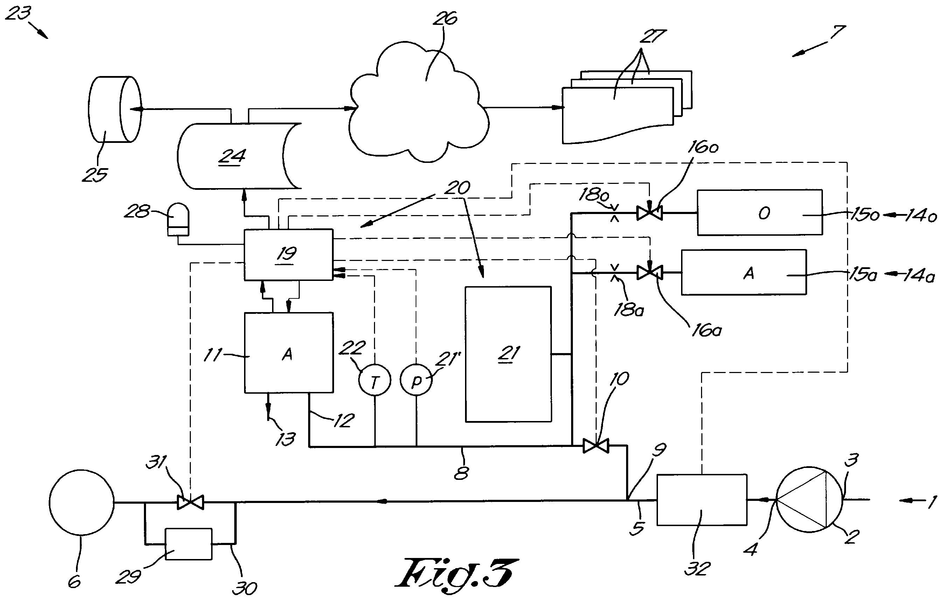

Figure 3 shows a variant embodiment of the device according to the invention, which differs from the device in figure 1 in that it is provided with means to generate an alarm signal in step 203, albeit for example visually or acoustically, when, during a monitoring period, the measurement results of an aforementioned measurement device 11 reach or exceed a set threshold value.

Furthermore, the compressed air network 1 of figure 3 is additionally provided with a treatment unit 29 downstream from the measuring line 8 that can be used to block impurities from the compressed air and that can be switched on or adjusted in the event of an alarm situation. Preferably, this is initiated automatically by control 19 when during a monitoring period the measurement results reach or exceed a set value.

The treatment unit 29 is, for example, a filter or dryer which is incorporated in a bypass line 30 of the supply line 5 and which can be switched on automatically by the control 19 by closing a bypass valve 31 in the supply line 5.

The device 1 of figure 3 is additionally provided in the supply line 5 with a unit 32 upstream from the branch of the measuring line 5 to block impurities, for example in the form of a filter or a dryer, and this unit 32 can preferably be automatically adjusted by the control in the case of a dryer or exchanged with a replacement unit in the case of a filter to lower the impurities during monitoring.

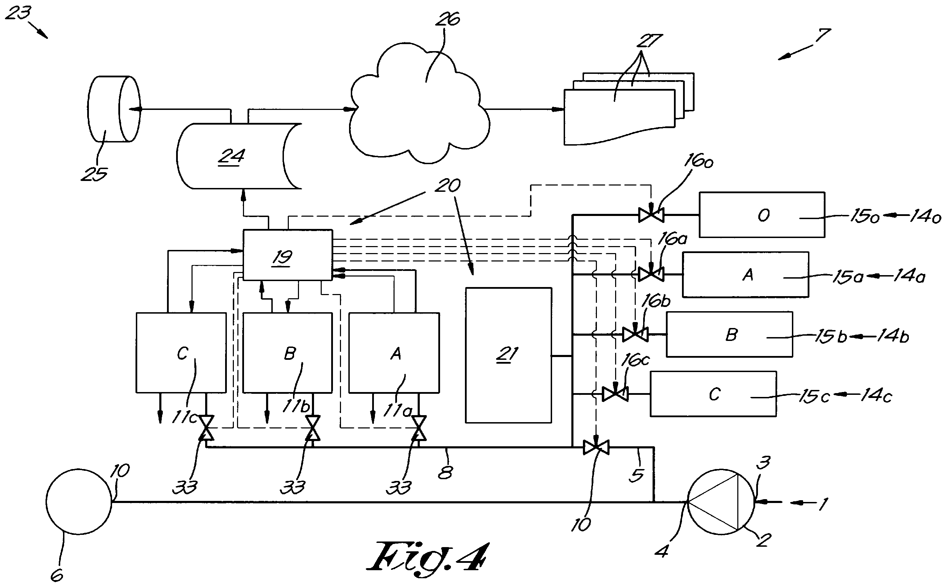

Figure 4 shows a device 7 according to the invention for monitoring, in this case, three different types of impurity, for example:
- type A i.e. the concentration of solid particles;
- type B i.e. the humidity; and
- Type C i.e. the concentration of oil, be it liquid form or in vapour form.

For each type of impurity, in addition to a gas cylinder 15o with zero gas, a gas cylinder 15a, 15b and 15c with a test gas of a known concentration of the type of impurity in question is provided.

For each type of impurity to be monitored, there is also a measuring device 11, respectively 11a, 11b and 11c, provided in the measuring line 8, each of which is provided with a selection valve 33 to selectively connect a corresponding measuring device 11 with its inlet to the measuring line 8 and selectively guide the gas flow in the measuring line 8 through one of the measuring devices 11a or 11b or 11c to the free atmosphere.

Such device 7 allows the compressed air to be monitored to be passed through the measuring line 8 to monitor continuously or periodically its quality by continuously measuring simultaneously the concentration of the three types of impurities and by periodically calibrating each of the three measuring devices 11b, 11c and 11d, either in sequence or simultaneously.

In the example each gas cylinder 15 contains a test gas with only one single known impurity. However, it cannot be excluded that two or three gas cylinders 15 containing a test gas may be replaced by one gas cylinder with a test gas containing two impurities, e.g. type A and B, each at a known concentration.

It cannot be excluded that, for a certain type of impurity, there may be several gas cylinders 15 containing test gases for that type of impurity, whereby each of these gas cylinders 15 contains a different concentration of the impurity in question.

It is clear that instead of continuous measurement, discrete measurements can also be carried out with a high frequency for monitoring the compressed air quality.

The present invention is by no means limited to the embodiments described as an example and shown in the figures, but such a device for monitoring the quality of the compressed air from a compressed air network and calibrating a measuring device used therein and method applied thereby can be realised according to different variants without departing from the scope of the invention.

## Claims

1. Device for monitoring the quality of the compressed air in a supply line of a compressed air network and calibrating a measuring device used for that purpose, more specifically a measuring device (11) for measuring the quantity of a specific type (A, B, C) of impurity in the compressed air, whereby the device is provided with:
- a measuring line (8, 8') which is intended to be connected to the supply line (5) and which is provided with a shut-off valve to close the measuring line (8, 8') of the supply line;
- an aforementioned measuring device (11) with an inlet for gas which is connected to the measuring line and a blow-off valve;
- means for supplying a test gas flow with a known impurity, more specifically with a known concentration or number of particles of the specific impurity (A, B, C), connected to the measuring line (8, 8') via a controllable shut-off valve (16) to enable the test gas to pass through it; **characterized in that** it is further provided
- a control (19) to which the aforementioned shut-off valves (10, 16) are connected to control these shut-off valves (10, 16) in such a way that either compressed air can be sent through the measuring device for monitoring the quality of the compressed air, or the aforementioned test gas can be sent through the measuring device to calibrate the measuring device.

2. Device according to claim 1, **characterised in that** the control (19) is programmed such, that:
• during the periods that the quality of the compressed air is monitored, the measuring line (8, 8') can be opened by the control (19) of the shut-off valves (10, 16) to allow the compressed air to be monitored flow through it for continuous or periodic measurement of the purity of the compressed air while the means for supplying a test gas are switched off; and
• during the calibration of the measuring device (11) the measuring line is isolated from the compressed air network and the measuring line (8, 8') is selectively and, in case of multiple test gases, alternately supplied with a test gas from a relevant gas cylinder (15) to calibrate the measuring device (11) by determining the deviation between the measured value and the known value of the concentration of the impurity of the relevant test gas.

3. Device according to claim 1 or 2, **characterised in that** the measuring device (11) is a measuring device, the measurement result of which is correctable and that the measuring device (11) is connected to the control (19) which is programmed for the calibration of the measuring device (11) to correct the measurement result of the measuring device (11) as a function of a determined deviation of the measurement result vis-à-vis the known value of the purity of the measured test gas.

4. Device according to any one of the previous claims, **characterised in that** it is provided with dosing means (20) to allow a dosed quantity of test gas to pass through the measuring line (8, 8') before each calibration measurement.

5. Device according to claim 4, **characterised in that** the dosing means (20) are formed by means (18) to determine the supplied gas flow of the test gas to the measuring line (8, 8') and by the control (19) which is programmed to open the shut-off valve (16) of the respective means of the test gas in doses.

6. Device according to any one of the previous claims, **characterised in that** the means for supplying a particular test gas flow are formed by a gas cylinder filled with such a test gas under pressure.

7. Device according to any one of the claims 1 to 5, **characterised in that** the means for supplying a particular test gas flow are formed by a gas cylinder filled with zero gas and a receptacle with a test liquid containing impurities of the measuring device to be calibrated (11) and by means to disperse the test liquid in the zero gas, for example by spraying or vaporising.

8. Device according to any one of the previous claims, **characterised in that** the means for supplying a test gas are means for supplying a test gas with only one single impurity, specifically intended for the calibration of a measuring device (11) intended for the measurement of this specific impurity.

9. Device according to any one of the previous claims, **characterised in that** the control (19) is programmed to periodically perform a calibration for each measuring device (11).

10. Device according to any one of the previous claims, **characterised in that** it is linked to a monitoring system (23) for collecting and recording the measurement data and calibration data of the measuring device (11) or measuring devices.

11. Device according to claim 10, **characterised in that** it is connected to a cloud service (26) with which the data of the monitoring system (23) can be recorded.

12. Compressed air network, **characterised in that** it contains a device according to any one of the previous claims for monitoring the quality of the compressed air from the compressed air network (1) and for automatically calibrating a measuring device (11) applied thereby

13. Compressed air network according to claim 12, **characterised in that** it is provided with means to indicate when, during a monitoring period, the measurement results of an aforementioned measuring device reach or exceed a set threshold.

14. Compressed air network according to claim 13, **characterised in that** downstream from the measuring line the compressed air network is provided with a treatment unit (29) that can be used to block impurities in the compressed air and that the control is such that when during a monitoring period the measurement results reach or exceed a set value, the unit is automatically switched on.

15. Method for monitoring the quality of the compressed air of a compressed air network (1) using at least one measuring device (11) to measure the concentration of a specific impurity and to periodically calibrate the measuring device used (11) connected to a measuring line, **characterised in that** the method contains the following steps:
- during the periods when the quality of the compressed air is monitored, branching off a compressed air flow to be monitored via the measuring line to pass it through the measuring device and continuously or periodically measuring the concentration of the relevant impurity in the compressed air to be monitored;
- during the periods of periodic calibration of the measuring device (11) used, isolating the measuring line with the measuring device from the compressed air system and performing the following steps:
• providing a test gas of a known concentration of the specific impurity for which the measuring device is calibrated and providing a test gas flow which is passed via the measuring line through the measuring device to be calibrated (11) ;
• measuring the concentration of this test gas and determining the deviation of the measured value compared to the known value of the test gas in question;
• where appropriate, correcting the measurement value of the measuring device (11) as a function of the detected deviations and/or recording the calibration data in a monitoring system (23).

16. Method according to claim 15, **characterised in that** it comprises the steps to calibrate successively with at least two test gases, namely with at least one impure test gas containing impurities of a known concentration or with a known number of particles of the specific impurity (A, B, C) and with a pure zero gas without this impurity.

## Patentansprüche

1. Vorrichtung zum Überwachen der Qualität der komprimierten Luft in einer Versorgungsleitung eines Kompressionsluftnetzes und Kalibrieren einer dazu verwendeten Messvorrichtung, insbesondere einer Messvorrichtung (11) zum Messen der Menge einer spezifischen Art (A, B, C) von Verunreinigung in der komprimierten Luft, wobei die Vorrichtung versehen ist mit:
- einer zum Verbinden mit der Versorgungsleitung (5) bestimmten Messleitung (8, 8'), die mit einem Absperrventil zum Schließen der Messleitung (8, 8') der Versorgungsleitung versehen ist;
- einer vorgenannten Messvorrichtung (11) mit einem Einlass für Gas, die mit der Messleitung und einem Abblasventil verbunden ist;
- Mittel zum Zuführen eines Prüfgasstroms mit einer bekannten Verunreinigung, insbesondere einer bekannten Konzentration oder Anzahl von Partikeln der spezifischen Verunreinigung (A, B, C), die über ein steuerbares Absperrventil (16) mit der Messleitung (8, 8') verbunden sind, um das Durchleiten des Prüfgases zu ermöglichen; **dadurch gekennzeichnet, dass** sie ferner mit Folgendem versehen ist
- einer Steuerung (19), mit der die vorgenannten Absperrventile (10, 16) verbunden sind, um diese Absperrventile (10, 16) derart anzusteuern, dass entweder komprimierte Luft zum Überwachen der Qualität der komprimierten Luft durch die Messvorrichtung geschickt werden kann, oder das vorgenannte Prüfgas zum Kalibrieren der Messvorrichtung durch die Messvorrichtung geschickt werden kann.

2. Vorrichtung nach Anspruch 1, **dadurch gekennzeichnet, dass** die Steuerung (19) derart programmiert ist, dass:
• während der Überwachungsperioden der Qualität der komprimierten Luft die Messleitung (8, 8') durch die Steuerung (19) der Absperrventile (10, 16) zum Hindurchströmen der zu überwachenden komprimierten Luft zur kontinuierlichen oder periodischen Messung der Reinheit der komprimierten Luft bei abgeschalteten Mitteln zum Zuführen eines Prüfgases geöffnet werden kann; und
• während der Kalibrierung der Messvorrichtung (11) die Messleitung vom Kompressionsluftnetz getrennt ist und die Messleitung (8, 8') selektiv und im Falle mehrerer Prüfgase abwechselnd mit einem Prüfgas aus einer betreffenden Gasflasche (15) versorgt wird, um die Messvorrichtung (11) durch Bestimmen der Abweichung zwischen dem Messwert und dem bekannten Wert der Konzentration der Verunreinigung des betreffenden Prüfgases zu kalibrieren.

3. Vorrichtung nach Anspruch 1 oder 2, **dadurch gekennzeichnet, dass** die Messvorrichtung (11) eine Messvorrichtung ist, deren Messergebnis korrigierbar ist, und dass die Messvorrichtung (11) mit der Steuerung (19) verbunden ist, die zum Kalibrieren der Messvorrichtung (11) programmiert ist, um das Messergebnis der Messvorrichtung (11) in Abhängigkeit von einer ermittelten Abweichung des Messergebnisses gegenüber dem bekannten Wert der Reinheit des gemessenen Prüfgases zu korrigieren.

4. Vorrichtung nach einem der vorhergehenden Ansprüche, **dadurch gekennzeichnet, dass** sie mit Dosiermitteln (20) versehen ist, um vor jeder Kalibrierungsmessung eine dosierte Prüfgasmenge durch die Messleitung (8, 8') zu leiten.

5. Vorrichtung nach Anspruch 4, **dadurch gekennzeichnet, dass** die Dosiermittel (20) durch Mittel (18) zum Bestimmen des zugeführten Gasstroms des Prüfgases zur Messleitung (8, 8') und durch die Steuerung (19), die programmiert ist, um das Absperrventil (16) der jeweiligen Mittel des Prüfgases dosiert zu öffnen, gebildet sind.

6. Vorrichtung nach einem der vorhergehenden Ansprüche, **dadurch gekennzeichnet, dass** die Mittel zum Zuführen eines bestimmten Prüfgasstroms durch eine mit einem solchen Prüfgas unter Druck gefüllte Gasflasche gebildet sind.

7. Vorrichtung nach einem der Ansprüche 1 bis 5, **dadurch gekennzeichnet, dass** die Mittel zum Zuführen eines bestimmten Prüfgasstroms durch eine mit Nullgas gefüllte Gasflasche und einen Behälter mit einer Verunreinigungen enthaltenden Prüfflüssigkeit der zu kalibrierenden Messvorrichtung (11) und durch Mittel zum Dispergieren der Prüfflüssigkeit in dem Nullgas, beispielsweise durch Zerstäuben oder Verdampfen, gebildet sind.

8. Vorrichtung nach einem der vorhergehenden Ansprüche, **dadurch gekennzeichnet, dass** die Mittel zum Zuführen eines Prüfgases Mittel zum Zuführen eines Prüfgases mit nur einer einzigen Verunreinigung sind, die speziell für die Kalibrierung einer Messvorrichtung (11) bestimmt ist, die für die Messung dieser spezifischen Verunreinigung bestimmt ist.

9. Vorrichtung nach einem der vorhergehenden Ansprüche, **dadurch gekennzeichnet, dass** die Steuerung (19) programmiert ist, um periodisch eine Kalibrierung für jede Messvorrichtung (11) durchzuführen.

10. Vorrichtung nach einem der vorhergehenden Ansprüche, **dadurch gekennzeichnet, dass** sie mit einem Überwachungssystem (23) zum Sammeln und Aufzeichnen der Messdaten und Kalibrierdaten der Messvorrichtung (11) oder der Messvorrichtungen verbunden ist.

11. Vorrichtung nach Anspruch 10, **dadurch gekennzeichnet, dass** sie mit einem Cloud-Dienst (26) verbunden ist, mit dem die Daten des Überwachungssystems (23) aufgezeichnet werden können.

12. Kompressionsluftnetz, **dadurch gekennzeichnet, dass** es eine Vorrichtung nach einem der vorhergehenden Ansprüche zum Überwachen der Qualität der komprimierten Luft aus dem Kompressionsluftnetz (1) und zum automatischen Kalibrieren einer von diesem beaufschlagten Messvorrichtung (11) enthält.

13. Kompressionsluftnetz nach Anspruch 12, **dadurch gekennzeichnet, dass** es mit Mitteln versehen ist, um anzuzeigen, wenn während einer Überwachungsperiode die Messergebnisse einer vorgenannten Messvorrichtung einen festgelegten Schwellenwert erreichen oder überschreiten.

14. Kompressionsluftnetz nach Anspruch 13, **dadurch gekennzeichnet, dass** das Kompressionsluftnetz stromabwärts der Messleitung mit einer Aufbereitungseinheit (29) versehen ist, mit der Verunreinigungen in der komprimierten Luft blockierbar sind, und dass die Steuerung derart ausgebildet ist, dass dann, wenn während einer Überwachungsperiode die Messergebnisse einen festgelegten Wert erreichen oder überschreiten, die Einheit automatisch eingeschaltet wird.

15. Verfahren zum Überwachen der Qualität der komprimierten Luft eines Kompressionsluftnetzes (1) unter Verwendung mindestens einer Messvorrichtung (11) zum Messen der Konzentration einer spezifischen Verunreinigung und zum periodischen Kalibrieren der verwendeten Messvorrichtung (11), die mit einer Messleitung verbunden ist, **dadurch gekennzeichnet, dass** das Verfahren folgende Schritte beinhaltet:
- während der Perioden, in denen die Qualität der komprimierten Luft überwacht wird, Abzweigen eines zu überwachenden Kompressionsluftstroms über die Messleitung, um ihn durch die Messvorrichtung zu leiten, und kontinuierliches oder periodisches Messen der Konzentration der betreffenden Verunreinigung in der zu überwachenden komprimierten Luft;
- während der Perioden der periodischen Kalibrierung der verwendeten Messvorrichtung (11), Trennen der Messleitung mit der Messvorrichtung vom Kompressionsluftsystem und Durchführen der folgenden Schritte:
• Bereitstellen eines Prüfgases mit einer bekannten Konzentration der spezifischen Verunreinigung, für die die Messvorrichtung kalibriert wird, und Bereitstellen eines Prüfgasstroms, der über die Messleitung durch die zu kalibrierende Messvorrichtung (11) geleitet wird;
• Messen der Konzentration dieses Prüfgases und Bestimmen der Abweichung des gemessenen Wertes gegenüber dem bekannten Wert des betreffenden Prüfgases;
• gegebenenfalls Korrigieren des Messwerts der Messvorrichtung (11) in Abhängigkeit von den erfassten Abweichungen und/oder Aufzeichnen der Kalibrierungsdaten in einem Überwachungssystem (23).

16. Verfahren nach Anspruch 15, **dadurch gekennzeichnet, dass** es die Schritte umfasst, um nacheinander mit mindestens zwei Prüfgasen zu kalibrieren, nämlich mit mindestens einem unreinen Prüfgas, das Verunreinigungen einer bekannten Konzentration oder eine bekannte Anzahl von Partikeln der spezifischen Verunreinigung (A, B, C) enthält, und mit einem reinen Nullgas ohne diese Verunreinigung.

## Revendications

1. Dispositif pour la surveillance de la qualité de l'air comprimé dans une ligne d'alimentation d'un réseau d'air comprimé et l'étalonnage d'un dispositif de mesure utilisé dans ce but, plus spécifiquement un dispositif de mesure (11) pour mesurer la quantité d'un type spécifique (A, B, C) d'impureté dans l'air comprimé, moyennant quoi le dispositif est pourvu de :
- une ligne de mesure (8, 8') qui est prévue pour être raccordée à la ligne d'alimentation (5) et qui est pourvue d'une vanne d'arrêt pour fermer la ligne de mesure (8, 8') de la ligne d'alimentation ;
- un dispositif de mesure susmentionné (11) avec une entrée pour un gaz qui est raccordé à la ligne de mesure et à une vanne de décharge ;
- des moyens pour alimenter un écoulement de gaz de test avec une impureté connue, plus spécifiquement avec une concentration ou un nombre connu(e) de particules de l'impureté spécifique (A, B, C), raccordés à la ligne de mesure (8, 8') par l'intermédiaire d'une vanne d'arrêt commandable (16) pour permettre au gaz de test de la traverser ; **caractérisé en ce que** il est en outre pourvu
- d'une commande (19) à laquelle les vannes d'arrêt susmentionnées (10, 16) sont connectées pour commander ces vannes d'arrêt (10, 16) d'une manière telle que soit de l'air comprimé peut être envoyé à travers le dispositif de mesure pour surveiller la qualité de l'air comprimé, soit le gaz de test susmentionné peut être envoyé à travers le dispositif de mesure pour étalonner le dispositif de mesure.

2. Dispositif selon la revendication 1, **caractérisé en ce que** la commande (19) est programmée de telle sorte que :
• pendant les périodes où la qualité de l'air comprimé est surveillée, la ligne de mesure (8, 8') peut être ouverte par la commande (19) des vannes d'arrêt (10, 16) pour permettre à l'air comprimé à surveiller de s'écouler à travers elle pour une mesure continue ou périodique de la pureté de l'air comprimé alors que les moyens pour alimenter un gaz de test sont coupés ; et
• pendant l'étalonnage du dispositif de mesure (11) la ligne de mesure est isolée du réseau d'air comprimé et la ligne de mesure (8, 8') est sélectivement et, en cas de multiples gaz de test, en alternance alimentée avec un gaz de test provenant d'une bouteille de gaz appropriée (15) pour étalonner le dispositif de mesure (11) en déterminant la déviation entre la valeur mesurée et la valeur connue de la concentration de l'impureté du gaz de test approprié.

3. Dispositif selon la revendication 1 ou 2, **caractérisé en ce que** le dispositif de mesure (11) est un dispositif de mesure, dont le résultat de mesure est corrigible et **en ce que** le dispositif de mesure (11) est connecté à la commande (19) qui est programmée pour l'étalonnage du dispositif de mesure (11) pour corriger le résultat de mesure du dispositif de mesure (11) en fonction d'une déviation déterminée du résultat de mesure vis-à-vis de la valeur connue de la pureté du gaz de test mesuré.

4. Dispositif selon l'une quelconque des revendications précédentes, **caractérisé en ce qu'il** est pourvu de moyens de dosage (20) pour permettre à une quantité dosée de gaz de test de traverser la ligne de mesure (8, 8') avant chaque mesure d'étalonnage.

5. Dispositif selon la revendication 4, **caractérisé en ce que** les moyens de dosage (20) sont formés par des moyens (18) pour déterminer l'écoulement de gaz alimenté du gaz de test à la ligne de mesure (8, 8') et par la commande (19) qui est programmée pour ouvrir la vanne d'arrêt (16) des moyens respectifs du gaz de test en doses.

6. Dispositif selon l'une quelconque des revendications précédentes, **caractérisé en ce que** les moyens pour alimenter un écoulement de gaz de test particulier sont formés par une bouteille de gaz remplie d'un tel gaz de test sous pression.

7. Dispositif selon l'une quelconque des revendications 1 à 5, **caractérisé en ce que** les moyens pour alimenter un écoulement de gaz de test particulier sont formés par une bouteille de gaz remplie de gaz zéro et d'un réceptacle avec un liquide de test contenant des impuretés du dispositif de mesure à étalonner (11) et par des moyens pour disperser le liquide de test dans le gaz zéro, par exemple par pulvérisation ou vaporisation.

8. Dispositif selon l'une quelconque des revendications précédentes, **caractérisé en ce que** les moyens pour alimenter un gaz de test sont des moyens pour alimenter un gaz de test avec une seule impureté unique, spécifiquement prévu pour l'étalonnage d'un dispositif de mesure (11) prévu pour la mesure de cette impureté spécifique.

9. Dispositif selon l'une quelconque des revendications précédentes, **caractérisé en ce que** la commande (19) est programmée pour mettre en œuvre périodiquement un étalonnage pour chaque dispositif de mesure (11).

10. Dispositif selon l'une quelconque des revendications précédentes, **caractérisé en ce qu'**il est lié à un système de surveillance (23) pour collecter et enregistrer les données de mesure et données d'étalonnage du dispositif de mesure (11) ou des dispositifs de mesure.

11. Dispositif selon la revendication 10, **caractérisé en ce qu'**il est connecté à un service en nuage (26) avec lequel les données du système de surveillance (23) peuvent être enregistrées.

12. Réseau d'air comprimé, **caractérisé en ce qu'**il contient un dispositif selon l'une quelconque des revendications précédentes pour surveiller la qualité de l'air comprimé provenant du réseau d'air comprimé (1) et pour étalonner automatiquement un dispositif de mesure (11) appliqué par celui-ci

13. Réseau d'air comprimé selon la revendication 12, **caractérisé en ce qu'**il est pourvu de moyens pour indiquer quand, pendant une période de surveillance, les résultats de mesure d'un dispositif de mesure susmentionné atteignent ou dépassent un seuil réglé.

14. Réseau d'air comprimé selon la revendication 13, **caractérisé en ce qu'**en aval de la ligne de mesure le réseau d'air comprimé est pourvu d'une unité de traitement (29) qui peut être utilisée pour bloquer les impuretés dans l'air comprimé et **en ce que** la commande est telle que lorsque pendant une période de surveillance les résultats de mesure atteignent ou dépassent une valeur réglée, l'unité est automatiquement activée.

15. Procédé de surveillance de la qualité de l'air comprimé d'un réseau d'air comprimé (1) en utilisant au moins un dispositif de mesure (11) pour mesurer la concentration d'une impureté spécifique et pour étalonner périodiquement le dispositif de mesure utilisé (11) raccordé à une ligne de mesure, **caractérisé en ce que** le procédé contient les étapes suivantes :
- pendant les périodes où la qualité de l'air comprimé est surveillée, la dérivation d'un écoulement d'air comprimé à surveiller par l'intermédiaire de la ligne de mesure pour le faire passer à travers le dispositif de mesure et la mesure continue ou périodique de la concentration de l'impureté appropriée dans l'air comprimé à surveiller ;
- pendant les périodes d'étalonnage périodique du dispositif de mesure (11) utilisé, l'isolement de la ligne de mesure avec le dispositif de mesure par rapport au système d'air comprimé et la mise en œuvre des étapes suivantes :
• fourniture d'un gaz de test d'une concentration connue de l'impureté spécifique pour laquelle le dispositif de mesure est étalonné et fourniture d'un écoulement de gaz de test que l'on fait passer par l'intermédiaire de la ligne de mesure à travers le dispositif de mesure à étalonner (11) ;
• mesure de la concentration de ce gaz de test et détermination de la déviation de la valeur mesurée par comparaison avec la valeur connue du gaz de test en question ;
• selon pertinence, correction de la valeur de mesure du dispositif de mesure (11) en fonction des déviations détectées et/ou enregistrement des données d'étalonnage dans un système de surveillance (23).

16. Procédé selon la revendication 15, **caractérisé en ce qu'**il comprend les étapes pour étalonner successivement avec au moins deux gaz de test, à savoir avec au moins un gaz de test impur contenant des impuretés d'une concentration connue ou avec un nombre connu de particules de l'impureté spécifique (A, B, C) et avec un gaz zéro pur dépourvu de cette impureté.
